# EUROPEAN PATENT APPLICATION

(11) **EP 1 852 067 A2**
(43) Date of publication of application: **07.11.2007**
(21) Application number: 07107392.8
(22) Date of filing: 03.05.2007
(51) Int. Cl.: A61B 5/11, A61F 13/42, A61F 13/15

(54) **Assembly of a diaper and a sensor**

(30) Priority: 03.05.2006 NL 1031745
(71) Applicant: Dansamar B.V., 2243 AM Wassenaar (NL)
(72) Inventor: Brilman, Arend Jan, 2243 AM, Wassenaar (NL)
(74) Representative: van Loon, C.J.J.

(57) **Abstract**

Assembly of a diaper (1) and a sensor (25) for at least determining a position of a user of the diaper, wherein the diaper is provided with an insertion opening (13) or coupling element (60) for coupling of the sensor to the diaper, such as Velcro. Method for forming a diaper, wherein from at least a top layer (28), a core material (29) and a bottom layer (30), at least partly a diaper is formed, such that the core material is locked between the top layer and the bottom layer, wherein the top layer is at least partly moisture-transmissive and during use is turned towards the body of a user and the bottom layer is substantially moisture-tight and during use is turned outwards, after which on the side of the diaper turned outwards during use an insertion opening or coupling element is provided.

## Description

This invention relates to an assembly of a diaper and a sensor for at least determining a position of the user of the diaper.

In EP 1023871 a sensor is described for registering the position of a child, for instance in a crib. By the use of this sensor, it can for instance be remotely determined whether a child is lying in a bed or crib in an undesirable manner. For instance, it can thus be established whether a child has come to lie in a prone position, which position might lead to crib death. In this publication of the same inventor, it is described that this sensor can be secured to an article of clothing of the child, for instance to a diaper. To that end, such sensor is provided with a clip with which it can be clamped over an upper longitudinal edge of the diaper.

An assembly of such a sensor and a diaper provides the advantage that attachment of the sensor is relatively simple, though it is not ideal. The object of the invention is to improve such an assembly.

In an assembly according to the invention, the diaper is provided with an insertion opening or coupling element for coupling of the sensor to the diaper.

In a first embodiment, the insertion opening or the coupling element is provided on the diaper by applying a strip of material thereon which is possibly partly clear of an outer surface of the diaper. In this way, on the outside of the diaper, in a particularly simple manner, an insertion opening or coupling element is created in which or to which the sensor can be coupled, for instance by insertion of the sensor and/or by slipping a clip of the sensor over the longitudinal edge of the clear part of the strip.

In a further embodiment, the strip is provided on the front side of the diaper, near an upper edge thereof, such that side flaps that are normally used for closing the diapers can be adhered thereon using tape, in particular in a reclosable manner.

In a second embodiment, the insertion opening or the coupling element is formed by applying a strip of material on the outside of the diaper, which strip consists of at least two layers, which are mutually connected adjacent two spaced apart ends and in a central portion are clear of each other, so that therebetween an opening is formed, while one of said layers is provided with an adhesive layer with which the strip is or can be adhered to the diaper.

The invention further relates to a method for forming a diaper, wherein from at least a top layer, a core material and a bottom layer, at least partly a diaper is formed, such that the core material is locked between the top layer and the bottom layer, wherein the top layer is at least partly moisture-transmissive and during use is turned to the body of a user and the bottom layer is substantially moisture-tight and during use is turned outwards, after which on the side of the diaper facing outwards during use an insertion opening or coupling element is provided.

In an embodiment, an insertion opening or coupling element is provided by gluing a strip of material at least partly onto a bottom layer turned outwards during use, such that in or by the strip the insertion opening is formed.

In a further embodiment, the strip is provided in such a manner that at the same time it can serve for securing material strips such as tape or Velcro with which in a usual manner a diaper can be closed around the body of a user, in particular in a reclosable manner, by securing side flaps connected with a dorsal part of the diaper, by means of such material strips onto said strip. Tape can then be, for instance, Velcro^{®} tape or adhesive tape.

The invention further relates to an assembly of a sensor and a receiver and/or base station, and/or the use of a sensor in a transmitter device such as a baby alarm.

To clarify the invention, embodiments of an assembly, diaper and sensor according to the invention will be further described with reference to the drawings, in which:
Fig. 1 shows a diaper according to the invention, in flat condition;
Fig. 2 shows a diaper according to the invention, in a condition of use, with a sensor;
Fig. 3 shows a strip of material according to the invention, in a first embodiment;
Fig. 4 shows a strip of material according to the invention, in a second embodiment;
Fig. 5 shows in partial cross section along the line V-V in Fig. 2 a diaper with insertion opening and sensor;
Fig. 6 schematically shows a number of steps in the production of a diaper with insertion opening;
Fig. 7 shows an assembly of a sensor, base station and receiver;
Fig. 8 shows a sensor with an adhesive strip; and
Fig. 9 shows in front and rear view a sensor with attachment means in the form of Velcro.

The embodiments given in the drawing and description are shown only by way of illustration and should not be construed as limiting in any way. In particular, combinations of components of the exemplary embodiments shown are also understood to be represented, though the description is not limited thereto. In this description, the same or corresponding parts have the same or corresponding reference numerals.

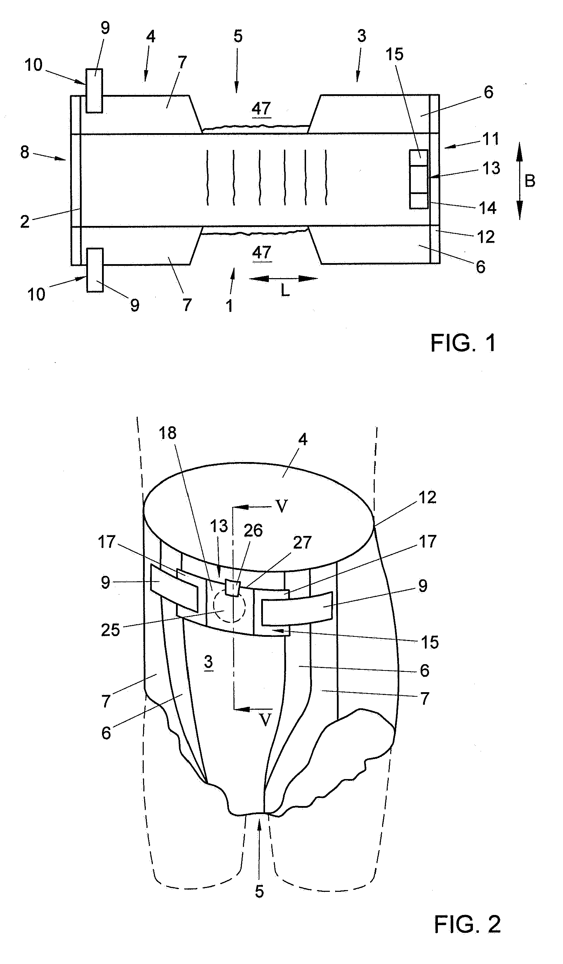

Fig. 1 schematically shows a diaper 1 in flat, unfolded condition, from the side facing outwards during use. This diaper 1 comprises a central part 2 with a ventral part 3, a dorsal part 4 and an intermediate crotch part 5. The central part 2 has a longitudinal direction L and a width direction B. The ventral part 3, viewed in the width direction B, is provided on opposite sides with preferably slightly elastic front side flaps 6; the dorsal part 4 is correspondingly provided with two preferably likewise elastic rear side flaps 7. The rear side flaps 7 are provided, near their edge 8 remote from the central part 2, with an adhesive strip 9 in the form of a strip of tape with an adhesive layer 10. The purpose thereof will be further elucidated hereinafter.

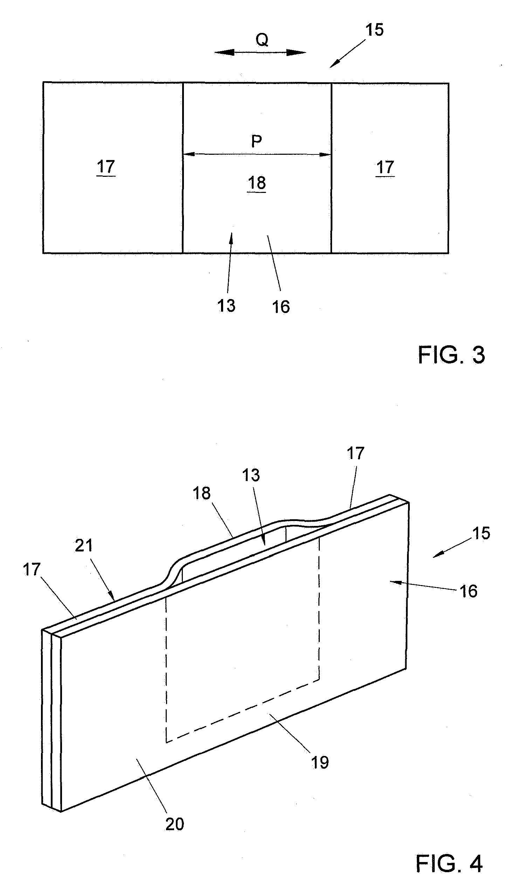

On the ventral part 3, near a longitudinal edge 11 which forms an upper band 12 during use, an insertion opening 13 is provided which, in this embodiment, is open at least on the upper side 14. This insertion opening has been formed on the diaper 1 by providing thereon a strip 15 of material, two examples of which are given in Figs. 3 and 4.

In Fig. 3, a strip 15 is provided, formed from one layer of synthetic material, for instance a plastic film. Suitable materials therefor will be immediately clear to those skilled in the art or be determined experimentally in a simple manner. In particular, for this, material can be used which is normally used for adhesive strips of diapers or on diapers for temporarily adhering such adhesive strips thereon. On the side 16 in use facing the diaper (the side shown in Figs. 3 and 4), the strip 15 is provided with two surfaces 17, spaced apart a distance p, which are provided with an adhesive layer or other adherent means. The intermediate part 18 of the strip is not provided with such an adhesive layer. This strip 15 has preferably been adhered to the diaper during production, for instance with a method to be further described hereinafter, such that the strip 15 has a longitudinal direction Q extending approximately parallel to the band 12 and slightly below it. The surfaces 17 are then fixedly connected with the diaper 1, while the intermediate part 18 lies clear thereof. In this way, between the diaper 1 (at least a bottom layer thereof) and the intermediate part 18 of the strip 15, an insertion opening 13 is formed, which is open in the direction of the band 12 (and in this embodiment also in the opposite direction).

In Fig. 4 an alternative embodiment of a strip 15 according to the invention is shown. Here, a first layer 20 and a second layer 21 are provided. The first layer is a strip of material which, on a side 16 in use facing the diaper 1, is provided with an adhesive layer or other adhering means, preferably over the entire surface. The second layer 21 is substantially designed as the strip 15 as shown in Fig. 3, which, however, has been secured onto the first layer 20 instead of onto the diaper. Here, therefore, the insertion opening 13 is formed between the first and second layers 20, 21. Such an embodiment can provide the advantage that the strip is easier to place on the diaper and moreover can lead to a more pleasant appearance.

Naturally, it is also possible, for instance, for a lower longitudinal edge of the central part 18 to be provided with an adhesive layer as well, so that this edge too is secured to the diaper or to the first layer 20, thus yielding a pocket-like insertion opening. This can simplify coupling of the sensor. Possibly, a stiffening can be co-formed, for instance along the upper side of the insertion opening 13, so that the insertion opening 13 is kept partly open or, conversely, is pressed into a closed condition. The stiffening can for instance be a strip of metal or plastic or a folded-over or thickened edge.

During use, as schematically shown in Fig. 2, the ventral part 3 will extend adjacent a user's belly, while the dorsal part 4 will extend adjacent a user's back. Clearly, the crotch part 5 extends between the legs of the user. The front closing flaps 6 have been folded over the hips, at least the upper legs, whereupon the rear closing flaps 7 have been folded over the front closing flaps 6 and secured with the tape 9. Visible on the front side of the diaper 1, on the ventral part 3, is the strip 15, adhered thereto with the surfaces 17. The tape 9 preferably engages the strip 15, in particular over the surfaces 17. A sensor 25 has been placed in the insertion opening 13, confined between the strip 15 and the diaper 1, in particular a bottom layer thereof. In this way, a coupling has been obtained. The sensor 25, shown in broken lines in Fig. 2, has a clip 26 which has been inserted over the upper longitudinal edge 27 of the insertion opening 13, over the strip 15, thereby yielding a better coupling still. The sensor 25 proper may then extend outside the insertion opening 13 while the clip has a free end reaching into the insertion opening and clamping onto the strip 15. If a strip 15 having a pocket-like insertion opening is used, the sensor 25 can be simply slipped into the insertion opening 13, possibly without clip 26, at least without clamping the clip over the strip 15. Also, at both the top and the bottom, a stiffening may be provided as mentioned above, so that the sensor 25 can be confined therebetween, or it can be clamped in the insertion opening. Also, other means may be provided for locking the sensor in the insertion opening, such as, for instance, a little flap which has been co-formed with the strip and can be closed over the insertion opening, for instance with an adhesive strip, Velcro or the like, or can be inserted with an insertion tab with the sensor 25 into the insertion opening. All this is understood to be at least covered by the designation coupling.

The strip 15, at least adjacent the parts situated opposite the surfaces 17, is preferably finished such that the tape 9 with which the rear closing flaps 7 are secured adhere well thereto and can preferably be pulled off and attached again several times, without damaging the diaper. In the known diapers, to that end, already material strips are stuck on the front side of the diaper. Such a material strip is suitable in particular for use on a diaper according to the invention, for it can be fitted simply, for instance with existing equipment, while only adhesion of a central part needs to be prevented. The material strip/strip 15 can for instance be provided with a suitable smooth surface, for instance of plastic, or, conversely, a Velcro adhering strip. Alternatively, other closing means may be provided, for instance press studs, clamps, clasps, or the like, although these make a diaper less simple to produce and more expensive.

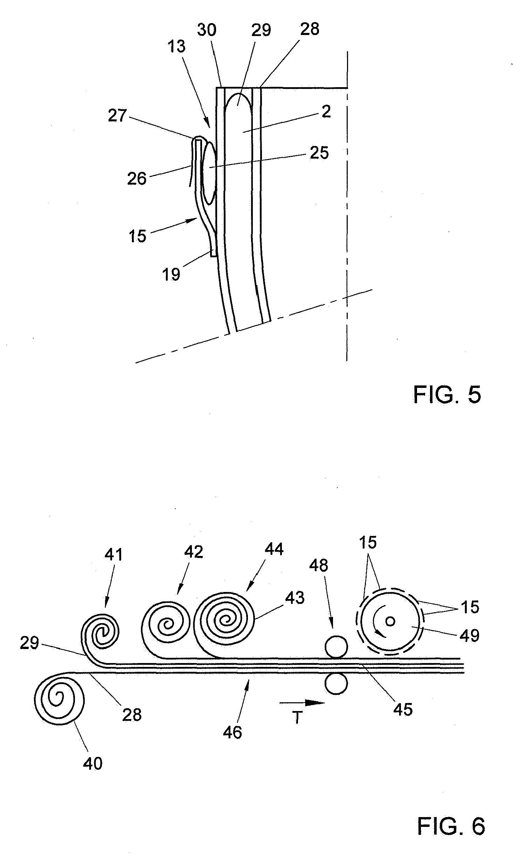

Fig. 5 shows, in sectional view along the line V-V in Fig. 2, a diaper 1 with sensor 25, in a simplified form. The diaper, apart from the insertion opening, can have any shape and structure known per se. Such aspects of diapers and their structure and method of manufacture are immediately clear to those skilled in the art and generally known. The diaper in Fig. 1, viewed from the top to the bottom, has a first layer 28, a core 29 of core material, in particular moisture absorbing material such as pulp, fibers and/or gel or the like, and a second layer 30. At least the first layer 28 and the second layer 30 are interconnected along the edges for forming an integral central part 2, thereby confining the core 29. The joint can be effected by sealing, gluing, stitching or any other suitable method. Side flaps 6, 7 (not shown in Fig. 5) are attached to the longitudinal edges or partly or wholly formed integrally with the top layer and/or bottom layer or other part of the diaper.

As is clearly visible in Fig. 5, an embodiment is represented here, in which a strip 15 has been attached, by the surfaces 17 and the lower longitudinal edge 19 of the intermediate part 18, to the bottom layer 30, for instance by gluing, stapling or sealing, thereby forming the insertion opening 13 in which the sensor 25 has been secured, for obtaining a coupling. To that end, the clip 26 has been slipped over the edge of the strip 15, so that the strip is clamped between the clip 26 and the sensor 25, and the sensor is thereby retained. It is preferred for this clamping to be such as to make it impossible for a child by itself to pull the clip off the strip or out of the insertion opening. Optionally, the clip may be designed such that, for instance, it needs to be unlocked first, for instance by a combined pulling and/or pushing and/or rotary movement, as in the case of a childproof closure of, for instance, a vial or other container, or that first a part needs to be removed, for instance a key-slot connection. Also, the clip may be so designed as to engage throughout the height of the strip, thus making removal still more difficult.

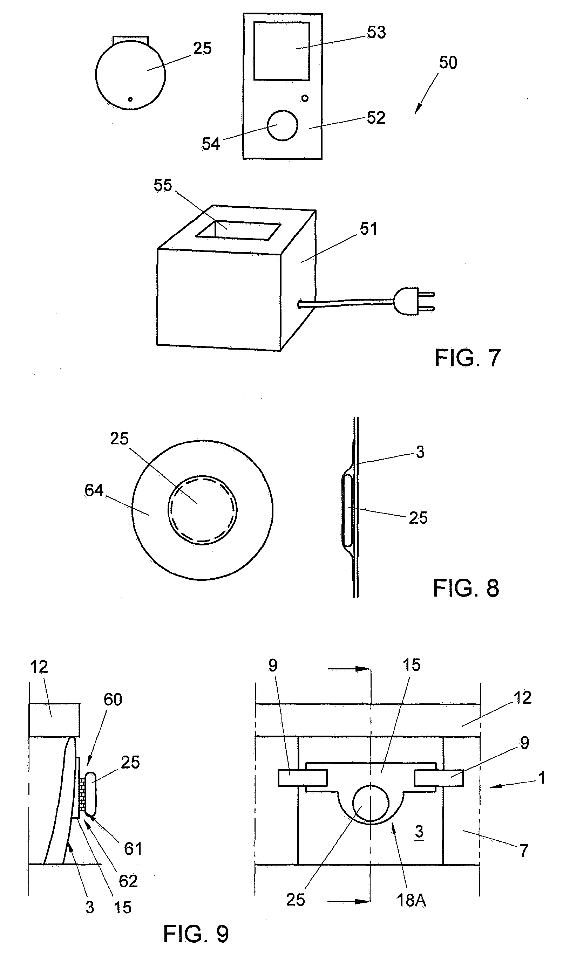

In Figs. 8 and 9, a sensor 25 is shown, secured to a band 15 on a diaper 1 by means of a Velcro fastening 60. To that end, one part 60 of the Velcro 60, for instance the hook part thereof, is attached against a rear side of the sensor 25, for instance permanently or with the aid of an adhesive layer, plaster, tape, removable glue or the like, or in the form of a sleeve or band in which or to which the sensor can be received or attached, while the other part 62, for instance the loop part, may be provided on the diaper 1 in the form of the band or strip 15, in particular centrally at the front as described earlier. By using a removable connection between Velcro and sensor, this part 61 of the Velcro can be replaced, for instance upon damage or (severe) fouling. Naturally, loop part and hook part can also be arranged with their position interchanged. The length of the strip 15 is preferably such that the material strips 9 which are here also of Velcro design (in particular as the part that is arranged on the sensor 25) can be secured on the strip 15 for reclosable closure of the diaper preferably in use and upon disposal. Velcro^{®} is conventionally also designated as hook and loop tape. Especially in this embodiment, it is advantageous when at least a central part 18A of the strip 15 has a height h₁ which is relatively great, preferably greater than usual, so as to provide sufficient space for placement of the sensor, in particular under or above the material strips 9 when the diaper is closed. The strip 15 forms a coupling element 63 for the sensor 25 and/or the material strips 9. It will be clear that as part of a Velcro connection 60, any material suitable for such a connection can be used, while, further, this may be a Velcro or part thereof that is already present on the diaper for (re)closability. To be understood by part of Velcro or a Velcro part is at least, though not exclusively so, one of the parts jointly suitable for forming a Velcro fastening, i.e. for instance the loop part or the hook part.

A diaper according to the invention has as an advantage that it is simple to manufacture, in some cases even with existing machines and with substantially the same structure. By the insertion opening 13 the advantage is achieved that the sensor 25 can always be arranged and held in the proper position, while the sensor can be reused again and again and the diaper can be of the disposable type. By fitting the sensor 25 slightly lower than the upper edge of the diaper, it does not hinder the user, or only minimally so, while moreover movements of the diaper relative to the body will be less pronounced there, so that the sensor can reflect the position of the user more accurately. Further, the user is not hindered by, for instance, a clip which presses over the band against the belly or back or other part of the body of the user. By providing the insertion opening on the ventral part 3 of the diaper 1, under the band thereof, it will extend adjacent the relatively thick and soft core 29, so that the hard parts of the sensor 25 are screened from the user. As a result, no irritation will occur, not even when the user proceeds to lie prone, while accurate measurement is still enabled at all times. Partly for that reason, the insertion opening 13 is preferably provided on the outside of the diaper 1, so that sensor 25 is screened off. Moreover, this effectively prevents fouling of the sensor by urine or feces. Further, in this way, the chances of the sensor being thrown away along with the diaper are reduced.

Fig. 6 schematically shows a portion of a method for manufacturing diapers 1 according to the invention. In it, from a first roller 40, a web of material for the first layer 28 is supplied, in flat condition, on which from a second roller 41 a strip of core material 29 is laid, over which, from a third roller 42, a strip of second layer 30 is laid. On opposite sides, viewed in transit direction T, a strip 43 of preferably slightly elastic material is supplied, also from a roller, 44. The longitudinal edges 45 of the first layer 28 and the second layer 30, together with an edge of the strip 43 situated thereon or therebetween, are interconnected, for instance by a sealing device 48. Next, on the bottom layer 30 a strip 15 is arranged which is secured by way of the surfaces 17 onto the bottom layer, for instance by gluing or sealing. This strip 15 can for instance be supplied such that it can be placed during transit of the web 46 of diaper material in the transit direction T, for which purpose for instance an application drum 49 or the like can be used. Further, from each strip 43, portions are removed so that in each case a front 6 and rear side flap 7 are formed with an intermediate leg opening 47. Furthermore, on each rear side flap 7 a piece of tape 9 is provided, with which the diaper 1 can be closed later. Comparable methods and equipment to be used therefor are known per se from the prior art, with the exception of at least the provision of the strip 15, such that it has an intermediate part 18 lying at least partly clear of the second layer 30 which faces outwards during use of the diaper 1. When the strip 15 is arranged in the same manner in the same position, with a known device which is also used in the prior art, the advantage is achieved that diaper manufacturers do not need to make any investments in new machines for forming a diaper according to the invention. Incidentally, it is of course also possible to use other methods and devices for forming diapers according to the invention.

In an alternative embodiment, a strip 15 is arranged on a diaper of the pants-type, such as a Pull-Up^{®} of the Procter & Gamble Company, USA. In that case, the strip is likewise provided on the outside of the diaper, preferably, though not necessarily so, before diaper pants are formed starting from a flat condition of the diaper. Such a pants-type diaper and a method for the manufacture thereof are given for instance in EP 0320991, but many variations thereon will be immediately clear to those skilled in the art.

Fig. 7 shows an apparatus 50 with a sensor 25, according to the invention. This sensor 25 is equipped with transmitting and receiving means for communication over a frequency band between 800 and 900 MHz, in particular a band with a frequency around 863 - 865 MHz. This band is a free band for transmission of radio signals, with a severe restriction on the allowable transmitting power. Moreover, this band is provided with a uniform protocol. Through the choice of this band, for the application as sensor for use with people, in particular young children, the advantage is achieved that negative radiation effects are avoided, because of the low power and the selected frequency, while a sufficiently large distance can be bridged between the sensor 25 and/or a receiving and transmitting station 51 (base station) and/or a portable receiver 52, for instance 50-200 m. Preferably, for a sensor 25, at least for use of the band on which the apparatus 50 works, no license needs to be paid, so that costs are kept relatively low. By the use of the protocol, the possibility is afforded of properly setting the sensitivity of the apparatus 51, so that disturbance by other installations is avoided. A further advantage of this band is that it is practically direction-insensitive. As a consequence, no complicated and/or costly and/or voluminous direction-sensitive transmitting and receiving means need to be used. An antenna, if any, can be simply built into the receiver 52 and/or the station 51.

A system as described with reference to Fig. 7 can be used particularly well as a baby alarm, precisely because of the low radiation. It consists in broad outline of a base station 51, one or more receivers 52 and possibly a part with the sensor 25. Between these parts, preferably coded, data are transmitted, as well as, preferably digitized, sound. Periodically, it is determined, by signal exchange, whether they are within mutual reach. Upon activation of the system, a recognition protocol is traversed, whereby it is established that the receiver 52, the station 51 and, possibly, the part with the sensor 25, match. This 'entry procedure' proceeds preferably entirely automatically, allowing only signals to be exchanged between these parts, without disturbance by other transmitting installations, while a user does not need to perform any complicated settings.

Preferably, the system is operated from the receiver, optionally allowing preferred settings. On the receiver, the status of the system and possibly of a person and/or object to be watched can be represented, preferably via a display 53 such as an LCD screen. This status can include information *inter alia* about: prone/supine position, at least the lying position of a person to be watched; within/beyond reach of the sensor 25 and/or the receiver 52 of the station 51; battery charge of the sensor 25 and/or the receiver 52; temperature in the room where the receiver 52 and/or the sensor 25 is/are situated; setting of sound intensity of the receiver 52 and/or the station 51. Preferably, an algorithm is provided, whereby in an alarm situation, for instance when prone position has been established with the sensor 25 as described earlier, a sound signal is produced which increases in volume and/or intensity with the lapse of time during which this situation occurs. Preferably, on the receiver 52 means 54 are provided with which this alarm signal can be switched off and possibly the apparatus can be turned off.

The or each receiver 52 can be recharged, preferably in a plug-in opening 55 in the base station, in a non-contact manner or otherwise, in a known manner.

In the sensor 25 an electronic or mechanical accelerometer may be provided with which periodically, for instance with a period of seconds or minutes, the position and/or movements of a child to be monitored can be registered. If these movements yield an indication that prone position seems (about) to occur, the period between the measurements will be shortened. Incidentally, it is of course also possible to register other movement patterns, for instance walking movements, sitting or lying and the like. If an undesired attitude or movement is registered, within a preselected period, an emergency signal is sent to the base station 51 and/or the receiver 52. If desired, it may be elected not to have the receiver 52 generate an emergency signal, so as not to startle and/or wake the child. It has been found that the selected frequency band is particularly advantageous precisely for baby alarm and baby monitoring functions because of the relatively low energy, good coding and transmission range.

As discussed, Fig. 8 shows an embodiment where a sensor 25 is provided with an adhesive strip 64 as a plaster or tape, with which the sensor 25 can be stuck onto a diaper or other article of clothing or possibly directly onto the skin. The sensor 25 can preferably be reused, while the adhesive strip can be discarded and be replaced with a new one. In the embodiment shown, the adhesive strip is designed as a strip of hook-and-loop fastener (Velcro^{®}) or at least one of the parts thereof, as shown in Fig. 9, with which it can be detachably adhered to an appropriate piece of material, for instance a slightly fibrous material or other (matching) counterpart of the Velcro, thus allowing simple placement and re-placement, as for instance a band or material strip already present for (re)closing the diaper.

The invention is not limited in any way to the embodiments represented in the drawing and description. Many variations thereon are possible within the concept of the invention.

For instance, other types of diapers can be used, or even other, disposable or non-disposable, articles of clothing, such as rompers, diaper overpants and the like. The insertion opening is preferably provided on the outside of the garment, preferably on a side being in front position during use, in particular at the level of the belly under the navel. However, of course other positions are also possible, as for instance on an inside of the garment or between a bottom layer and a top layer of a diaper, while diapers according to the invention can also have a different makeup, for instance with more layers, other closing means, other shapes and the like. A sensor 25 in particular and an apparatus 50 in general may be designed differently and may have additional functions, such as a baby alarm function, a tether function, whereby a signal is produced if the user with the sensor moves beyond a preset distance from a base station 51 and/or the receiver 52, while preferably at least on the receiver 52 an (alarm) signal is represented and, incidentally, the possibility of display of the measured distance may be provided. Further, a motion sensor function, for instance for step counting, walking movement registration or the like may be provided. A sensor 25 according to the invention is preferably cleanable, for instance washable in a washing machine. Alternatively, the sensor 25 can be made of disposable design, for instance in cheap chip technology, such as RFID chips or transponder technology. An insertion opening can be formed in a different way, for instance by a cut in an (outer) layer of the diaper through which the sensor or a clip or the like can be inserted, which cut is for instance closable with a strip, tape or the like. Preferably, however, in that case a lining is provided, such that the sensor cannot move in the core, which would render it difficult to remove. Also, a strip of material can terminate at a distance from the band 12, so that the sensor can be clipped onto it. In all designs, an insertion opening can also at least functionally be seen as a coupling element or vice versa.

## Claims

1. An assembly of a diaper and a sensor for at least determining a position of a user of the diaper, wherein the diaper is provided with an insertion opening or coupling element for coupling of the sensor to the diaper.

2. An assembly according to claim 1, wherein the insertion opening or the coupling element is formed on or near a centerline of the diaper, which centerline extends from a front side of the diaper as far as a rear side of the diaper.

3. An assembly according to any one of the preceding claims, wherein the insertion opening or the coupling element is provided on a front side of the diaper.

4. An assembly according to any one of the preceding claims, wherein the insertion opening or the coupling element is provided on an outer side of the diaper, in particular by providing a strip of material on an outer surface of the diaper.

5. An assembly according to claim 4, wherein the strip has two mutually spaced apart contact surfaces by which the strip is secured onto the diaper, while between the contact surfaces there is at least one strip part that is clear of the diaper.

6. An assembly according to claim 4 or 5, wherein the strip is provided on the diaper near an operatively upper longitudinal edge of a ventral part, while the diaper is provided with two side flaps with tape, which are connected with a dorsal part, which tape can be adhered to the strip for closing the diaper.

7. An assembly according to claim 4 or 5, wherein the strip is at least partly designed as a part of a Velcro fastening, wherein the sensor and possibly two side flaps connected with a dorsal part are provided with a component of the Velcro fastening for the purpose of securing the sensor and closing the diaper, respectively.

8. An assembly according to any one of the preceding claims, wherein the insertion opening is open on two opposite sides.

9. An assembly according to any one of the preceding claims, wherein the insertion opening is in the form of a pocket.

10. An assembly according to any one of the preceding claims, wherein the sensor is receivable in the insertion opening or the coupling element.

11. An assembly according to any one of the preceding claims, wherein at least the sensor is provided with means for securing it to or in the insertion opening or the coupling element.

12. An assembly according to claim 11, wherein said means are clamping means and/or sticking means.

13. An assembly according to any one of the preceding claims, wherein the sensor is provided with transmitter means, wherein furthermore receiver means are provided which can communicate with said transmitter means on a band with a frequency between 800 and 900 MHz, more particularly between 860 and 875 MHz and preferably between 863 and 865 MHz.

14. A diaper, suitable and intended for use in an assembly according to any one of the preceding claims.

15. A strip of material, suitable and intended for use in an assembly according to any one of claims 1-12 or for forming a diaper according to claim 14.

16. A sensor for use in an assembly according to any one of claims 1-13 or with a diaper according to claim 14.

17. A method for forming a diaper, wherein from at least a top layer, a core material and a bottom layer, at least partly a diaper is formed, such that the core material is locked between the top layer and the bottom layer, wherein the top layer is at least partly moisture-transmissive and during use is turned towards the body of a user and the bottom layer is substantially moisture-tight and during use is turned outwards, after which on the side of the diaper turned outwards during use an insertion opening or coupling element is provided.

18. A method according to claim 17, wherein the insertion opening or the coupling element is provided by attaching, in particular gluing, a strip of material onto the diaper.

19. A method according to claim 17 or 18, wherein the insertion opening or the coupling element is provided on the diaper part when the top layer and the bottom layer lie substantially flat and side flaps near a front side of the diaper are not connected with side flaps near the rear side of the diaper other than by a central area of the diaper through which extends the core material.

20. A method according to any one of claims 17-19, wherein a coupling element is provided in the form of a Velcro part.
